# EUROPÄISCHE PATENTANMELDUNG

(11) **EP 0 778 265 A1**
(43) Veröffentlichungstag der Anmeldung: **11.06.1997**
(21) Anmeldenummer: 96118889.3
(22) Anmeldetag: 25.11.1996
(51) Int. Cl.: C07C 227/40, C07C 231/22

(54) **Verfahren zur Herstellung hellfarbiger Betaine**

(30) Priorität: 04.12.1995 DE 19545134
(71) Anmelder: Henkel Kommanditgesellschaft auf Aktien, 40589 Düsseldorf-Holthausen (DE)
(72) Erfinder: Pi Subirana, Rafael, Dr., 08400 Granollers (ES); Prat Queralt, Ester, Dr., 08238 Alella (ES); Bigorra Llosas, Joaquim, Dr., 08203 Sabadell (ES)

(57) **Zusammenfassung**

Es wird ein neues Verfahren zur Herstellung zur Herstellung hellfarbiger Betaine vorgeschlagen, bei dem man die Rohprodukte nach der Kondensation von Aminverbindungen mit Halogencarbonsäuren bzw. deren Salzen mit Hydrogensulfiten behandelt. Die Produkte sind nicht nur hellfarbig, sondern erweisen sich auch bei längerer Lagerung bei erhöhten Temperaturen als ausgesprochen farbstabil.

## Beschreibung

### Gebiet der Erfindung

Die Erfindung betrifft ein Verfahren zur Herstellung hellfarbiger Betaintenside durch Nachbehandlung mit Hydrogensulfiten.

### Stand der Technik

Betaine bzw. amphotere Tenside sind ausgesprochen hautverträglich und weisen ausgezeichnete Reinigungseigenschaften auf. Sie eignen sich daher in besonderer Weise zur Konfektionierung einer Vielzahl von oberflächenaktiven Produkten. Zu ihrer Herstellung geht man im einfachsten Fall von tertiären Aminen aus, die mit Natriumchloracetat zu Alkylbetainen umgesetzt werden. Die Umsetzung von Fettsäureaminoamiden oder Imidazolinen mit Natriumchloracetat führt zur Bildung von amphoteren Tensiden vom Typ der Glycinate; wird als Alkylierungsmittel Acrylsäureester eingesetzt, bilden sich Aminopropionate. Verbindungen der genannten Art sind in einer Vielzahl von Übersichtsartikeln beschrieben, von denen an dieser Stelle nur **Parf.Cosm.Arom. 70, 67 (1986), HAPPI, 70, (Nov.1986)** und **Soap Cosm.Chem.Spec. 46, (Apr.1990)** genannt sein sollen.

Ein Problem bei der Herstellung von Betainen besteht in ihrer unzureichenden Farbqualität bzw. Farbstabilität bei Temperaturbelastung. Im Gegensatz zu anderen beispielsweise anionischen Tensiden, deren Farbe sich durch Peroxid- oder Hypochloritbehandlung dauerhaft aufhellen läßt, versagen diese Verfahren im Fall der Betaine. Bei der Behandlung mit Wasserstoffperoxid werden sogar Produkte erhalten, die bei Lagerung bei erhöhter Temperatur eine stärkere Verfärbung zeigen, als unbehandelte Produkte.

Die Aufgabe der Erfindung hat somit darin bestanden, ein Verfahren zur Herstellung von hellfarbigen Betainen zur Verfügung zu stellen, das frei von den geschilderten Nachteilen ist. Insbesondere sollten Produkte einer ausreichenden Farbqualität und gleichzeitig Farbstabilität erhalten werden.

### Beschreibung der Erfindung

Gegenstand der Erfindung ist ein Verfahren zur Herstellung zur Herstellung hellfarbiger Betaine durch Kondensation von Aminverbindungen mit Halogencarbonsäuren bzw. deren Salzen, bei dem man die Betaine nach Abschluß der Kondensation mit Hydrogensulfiten behandelt.

Überraschenderweise wurde gefunden, daß die Nachbehandlung der Betainrohprodukte nach Abschluß der Kondensationsreaktion schon bei Einsatz sehr geringer Mengen an Hydrogensulfiten nicht nur zu einer deutlichen Farbaufhellung führt, sondern daß die Produkte im Vergleich mit den Verfahrens des Stands der Technik auch im Verlauf der Lagerung bei höheren Temperaturen eine deutlich höhere Farbstabilität aufweisen. Besonders vorteilhaft ist in diesem Zusammenhang die Kombination aus Peroxidbleiche und Hydrogensulfitnachbehandlung.

### Aminverbindungen

Aminverbindungen, die im Sinne des erfindungsgemäßen Verfahrens in Betracht kommen, folgen der Formel **(I),** 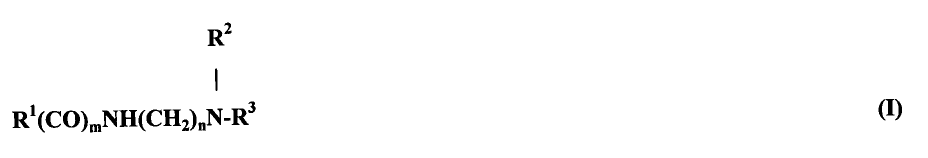 in der R¹(CO)ₘ für einen aliphatischen Acyl- bzw. Alkylrest mit 8 bis 22 Kohlenstoffatomen, R² und R³ unabhängig voneinander für einen Alkylrest mit 1 bis 4 Kohlenstoffatomen und m für 0 oder 1 und n für Zahlen im Bereich von 1 bis 5 steht. Demzufolge stellen die Aminverbindungen entweder Diamine oder Fettsäureaminoamide dar. Typische Beispiele hierfür sind insbesondere Kokosfettsäureaminoamide, in denen R¹CO für einen Acylrest mit 8 bis 18 und insbesondere 12 bis 14 Kohlenstoffatomen, n für 2 oder 3 und R² und R³ für Methylgruppen stehen.

### Halogencarbonsäuren

Die in Frage kommenden Halogencarbonsäuren bzw. deren Salze folgen der Formel **(II),** 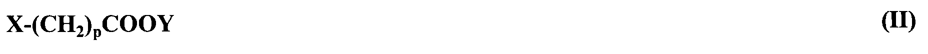 in der X für ein Halogen, Y für Wasserstoff oder ein Alkalimetall und p für Zahlen im Bereich von 1 bis 5 steht. Vorzugsweise wird für die Kondensation Natriumchloracetat eingesetzt.

### Hydrogensulfite

Als geeignete Hydrogensulfite kommen Alkalihydrogensulfite wie beispielsweise Kalium- oder Ammoniumhydrogensulfit in Frage. Bevorzugt ist die Verwendung von Natriumhydrogensulfit. Die Alkalihydrogensulfite können in Mengen von 0,005 bis 1 und vorzugsweise 0,01 bis 0,5 Gew.-% - bezogen auf die Betaine - eingesetzt werden. Hierbei ist es unkritisch, ob die Hydrogensulfite in fester Form oder als wäßrige Lösungen eingesetzt werden.

### Kondensation und Bleiche

Die Durchführung der Kondensationsreaktion erfolgt in an sich bekannter Weise, wobei man das Fettsäureaminoamid, vorzugsweise Kokosfettsäureaminoamide mit 8 bis 18 bzw. 12 bis 14 Kohlenstoffatomen im Fettacylrest, und die Halogencarbonsäuren bzw. deren Salze, vorzugsweise Natriumchloracetat, unter Einhaltung eines pH-Wertes im Bereich von 7 bis 8 erhitzt und nachdem der Gehalt an freiem Aminoamid unter 0,5 Gew.-% abgesunken ist, die Reaktionsmischung in einem Druckgefäß über einen Zeitraum von 1 bis 2 h einer Nachbehandlung bei einer Temperatur von 100 bis 130°C und einem pH = 10 bis 14 unterwirft. Nach Abschluß der Nachreaktion empfiehlt es sich, das Konzentrat wieder auf einen neutralen pH-Wert einzustellen.

Zur Herstellung hochkonzentrierter, niedrigviskoser Betaine mit einem Feststoffgehalt im Bereich von 40 bis 60 Gew.-% empfiehlt es sich, die Kondensationsreaktion in Gegenwart von Polyolen wie beispielsweise Glycerin oder Sorbitol, Alkylpolyglucosiden, Fettsäurepartialglyceriden, Fettalkoholethoxylaten, Glycolsäure, Sulfobetainen und dergleichen durch-zuführen. Entsprechende Verfahren sind aus den Druckschriften **DE-A1 43 08 793, DE-C1 43 37 324, DE-C1 43 40 423** und **DE-C1 44 08 183** (Henkel) bekannt, auf deren Lehre hiermit ausdrücklich Bezug genommen wird. Weiterhin vorteilhaft ist die Verwendung von Fettsäuren bzw. Seifen in Mengen von 0,5 bis 3 Gew.-% - bezogen auf die Produkte.

Die Bleiche schließt sich üblicherweise an die Kondensation an. Es ist jedoch auch möglich, Fertigprodukte im Anschluß an die alkalische Nachbehandlung zu bleichen. In der Regel wird den Rohbetainen eine entsprechende Menge Hydrogensulfit, vorzugsweise Natriumhydrogensulfit entweder als Pulver oder aber als wäßrige Lösung zugegeben. Die Bleiche findet dann über einen Zeitraum von 1 bis 5, vorzugsweise 2 bis 3 Stunden bei Temperaturen im Bereich von 50 bis 90 und vorzugsweise um 60 bis 70°C statt. In einer besonderen Ausführungsform der Erfindung wird den Rohbetainen zunächst eine Menge von 0,5 bis 5, vorzugsweise 1 bis 2 Gew.-% Wasserstoffperoxid, beispielsweise in Form einer 25 bis 35 Gew.-%ogen Lösung zugesetzt. Nach der Vorbleiche erfolgt die Zugabe von Hydrogensulfit in der oben beschriebenen Weise.

### Gewerbliche Anwendbarkeit

Die nach dem erfindungsgemäßen Verfahren erhältlichen Betaine sind hellfarbig und lagerstabil und eignen sich daher zur Herstellung einer Vielzahl von oberflächenaktiven Mitteln wie beispielsweise Handgeschirrspülmitteln und Haarshampoos, in denen sie in Mengen von 0,5 bis 35, vorzugsweise 1 bis 10 Gew.-% - bezogen auf die Mittel - enthalten sein können. Diese Mittel können eine Vielzahl von Hilfs- und Zusatzstoffen enthalten, als da beispielsweise sind: Emulgatoren, Überfettungsmittel, Verdickungsmittel, Kationpolymere, Siliconverbin-dungen, biogene Wirkstoffe, Filmbildner, Konservierungsmittel sowie Farb- und Duftstoffe. Werden Konservierungsmittel vom Typ des Kathon® CG eingesetzt, empfiehlt es sich, die Produkte durch Zusatz von 0,5 bis 2 Gew.-% - bezogen auf die Mittel - eines Alkalibromats, vorzugsweise Kaliumbromat, zu stabilisieren.

### Beispiele

**Allgemeine Hersteilvorschrift für Betaine.** 227,2 g (1,95 mol) Natriumchloracetat wurden in ca. 1400 ml Wasser gelöst. Anschließend wurden bei 40°C 55,3 g (0,19 mol) eines Fettsäureaminoamids eingerührt, das durch Amidierung einer gehärteten Kokosfettsäure mit 3-N,N-Dimethylaminopropylamin hergestellt worden war und einen Gehalt von 4,6 Gew.-% titrierbaren Stickstoff aufwies. Nach Erwärmen des Gemisches auf 90°C und Bildung einer klaren, dünnflüssigen Lösung wurden weitere 497,5 g (1,71 mol) des Fettsäureaminoamids zugegeben. Unter Einhaltung eines pH- Wertes zwischen 7,5 und 9,0 wurde die Reaktion fortgesetzt, bis nach ca. 2 h der Gehalt an freien Aminfunktionen unter 0,5 mmol/ 100 g, d.h. < 0,15 % Fettsäureaminoamid (ermittelt durch HPLC-Analyse) abgesunken war. Dem Ansatz wurden nun entsprechend einem pH-Wert von 12,5 in 10 Gew.-%iger Produktlösung 43,2 g 37 Gew.-%ige Natronlauge zugesetzt und das Produkt in einer Druckapparatur 1 h bei 120°C gerührt. Nach der Abkühlung wurde der Produkt-pH-Wert mit 53,8 g 24-Gew.- %iger Salzsäure auf 7,0 eingestellt.

### Beispiel 1, Vergleichsbeispiele V1 und V2

Entsprechend der allgemeinen Vorschrift wurde der Reaktionsansatz geteilt. Ein Drittel des Rohbetains wurde mit 0,02 Gew.-% Natriumhydrogensulfit versetzt und 1h bei 70°C gerührt. Anschließend wurden der nachbehandelte Ansatz (erfindungsgemäßes Beispiel 1) und der unbehandelte Ansatz (Vergleichsbeispiel V1) über einen Zeitraum von 1 bis 53 Tagen bei 30 bzw. 40°C gelagert und die Farbzahlen (APHA) ermittelt. Als weiteres Vergleichsbeispiel wurde ein weiteres Drittel des Ansatzes mit 0,04 Gew.-% Wasserstoffperoxid (35 Gew.-%ige wäßrige Lösung, V2) versetzt und 1 h bei 70°C gebleicht. Die Ergebnisse sind in Tabelle 1 zusammengefaßt.

**Tabelle 1**

| **Bleiche mit/ohne Hydrogensulfit** | | | | | | |
|---|---|---|---|---|---|---|
| **Bsp.** | **Verfahren** | **Farbzahl (APHA) nach Lagerzeit (20°C/40°C)** | | | | |
| | | **1 d** | **8 d** | **16 d** | **29 d** | **53 d** |
| 1 | mit Hydrogensulifit | 17/17 | 16/17 | 20/28 | 30/35 | 22/32 |
| V1 | ohne Nachbehandlung | 42/42 | 31/41 | 37/58 | 43/75 | 38/75 |
| V2 | mit Wasserstoffperoxid | 42/42 | 40/100 | 40/175 | 40/175 | 40/180 |

Die Beispiele und Vergleichsbeispiele zeigen, daß durch die Nachbehandlung mit Hydrogensulfit Betaine erhalten werden, die sich durch eine hellere Farbe und eine verbesserte Farbstablität auszeichnen. Demgegenüber führt eine konventionelle Wasserstoffperoxidbleiche nur zu wenig aufgehellten Produkten, die sich bei höherer Lagertemperatur sehr rasch dunkel verfärben.

### Beispiel 2, Vergleichsbeispiel V3

Es wurde ein Betain gemäß der allgemeinen Versuchsvorschrift hergestellt. Nach Abschluß der Kondensation zwischen der Aminverbindung und dem Natriumchloracetat wurde ein pH-Wert von 10 eingestellt und der Ansatz geteilt. Die eine Hälfte wurde bei einer Temperatur von 70°C über einen Zeitraum von 3 h portionsweise mit 1,2 Gew.-% - bezogen auf das Rohprodukt - Wasserstoffperoxid in Form einer 35 Gew.-%igen Lösung versetzt. Temperatur und pH-Wert wurden über weitere 2,5 h aufrechterhalten, bis kein Peroxid mehr nachzuweisen war. Anschließend wurden 0,01 Gew.-% - bezogen auf das Rohprodukt - Natriumhydrogensulfit zugegeben und eine weitere Stunde gerührt. Anschließend wurde das Betain durch Zugabe von verdünnter Salzsäure auf pH = 6,5 eingestellt (erfindungsgemäßes Beispiel 2). Die andere Hälfte wurde wie oben angegeben nur unter Zugabe von Wasserstoffperoxid gebleicht (Vergleichsbeispiel V3). Schließlich wurden beide Produkte bei 20 bzw. 40°C über einen Zeitraum von 52 Tagen gelagert und die Farbzahlen (APHA) ermittelt. Die Ergebnisse sind in Tabelle 2 zusammengefaßt:

**Tabelle 2**

| **Bleiche mit/ohne Hydrogensulfit** | | | | | | |
|---|---|---|---|---|---|---|
| **Bsp.** | **Bleiche** | **Farbzahl (APHA) nach Lagerzeit (20°C/40°C)** | | | | |
| | | **1 d** | **7 d** | **15 d** | **28 d** | **52 d** |
| 2 | H₂O₂/Hydrogensulifit | 14/14 | 6/16 | 18/26 | 18/25 | 18/25 |
| V3 | H₂O₂ | 17/17 | 16/29 | 30/42 | 30/48 | 31/48 |

Das Beispiel und das Vergleichsbeispiel zeigen, daß durch den Zusatz von Hydrogensulfit nach der Peroxidbleiche eine verbesserte Farbstabilität bei Lagerung bei erhöhter Temperatur erzielt wird.

## Patentansprüche

1. Verfahren zur Herstellung zur Herstellung hellfarbiger Betaine durch Kondensation von Aminverbindungen mit Halogencarbonsäuren bzw. deren Salzen, **dadurch gekennzeichnet,** daß man die Betaine nach Abschluß der Kondensation mit Hydrogensulfiten behandelt.

2. Verfahren nach Anspruch 1, **dadurch gekennzeichnet**, daß man Aminverbindungen der Formel (I) einsetzt, 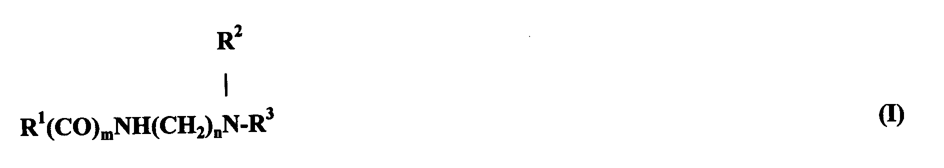 in der R¹(CO)ₘ für einen aliphatischen Acyl- bzw. Alkylrest mit 8 bis 22 Kohlenstoffatomen, R² und R³ unabhängig voneinander für einen Alkylrest mit 1 bis 4 Kohlenstoffatomen und m für 0 oder 1 und n für Zahlen im Bereich von 1 bis 5 steht.

3. Verfahren nach den Ansprüchen 1 und 2, **dadurch gekennzeichnet**, daß man Halogencarbonsäuren bzw. deren Salze der Formel **(II)** einsetzt, 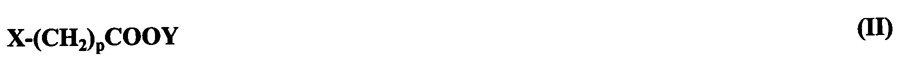 in der X für ein Halogen, Y für Wasserstoff oder ein Alkalimetall und p für Zahlen im Bereich von 1 bis 5 steht.

4. Verfahren nach den Ansprüchen 1 bis 3, **dadurch gekennzeichnet**, daß man die Hydrogensulfite in Mengen von 0,005 bis 1 Gew.-% - bezogen auf die Betaine - einsetzt.

5. Verfahren nach den Ansprüchen 1 bis 4, **dadurch gekennzeichnet,** daß man Natriumhydrogensulfit einsetzt.

6. Verfahren nach den Ansprüchen 1 bis 5, **dadurch gekennzeichnet**, daß man die Betaine zunächst mit Wasserstoffperoxid und anschließend mit Hydrogensulfiten behandelt.
